# FASCICULE DE BREVET EUROPEEN

(11) **EP 3 829 437 B1**
(45) Date de publication et mention de la délivrance du brevet: **06.11.2024**
(21) Numéro de dépôt: 19749347.1
(22) Date de dépôt: 02.08.2019
(51) Int. Cl.: A61B 5/0531, A61B 5/157, A61B 5/00, A61B 5/053, A61B 5/06, A61B 5/15, A61B 5/145

(54) **GESTION ENFONCEMENT DE MICROAIGUILLES**
MIKRONADELEINDRINGUNGSMANAGEMENT
MICRONEEDLE INDENTATION MANAGEMENT

(30) Priorité: 03.08.2018 FR 1857291
(43) Date de publication de la demande: 09.06.2021
(73) Titulaire: WIZP AS, 3183 Horten (NO)
(72) Inventeur: PIERART, Luc, 94800 VILLEJUIF (FR); LÊ, Anh-Minh, 92200 Neuilly-sur-Seine (FR)
(74) Mandataire: Regimbeau
(86) Numéro de dépôt international: PCT/EP2019/070947
(87) Numéro de publication internationale: WO 2020/025822

(56) Documents cités:
- WO-A1-2014/120114
- US-A1- 2014 275 897
- US-A1- 2015 208 984
- US-A1- 2017 347 925

## Description

### DOMAINE TECHNIQUE GENERAL

La présente invention concerne un dispositif de surveillance corporelle via analyse de liquide corporel, typiquement interstitiel, à l'aide de micro-aiguilles.

Plus précisément, la présente invention concerne la gestion du maintien des micro-aiguilles dans la peau.

### ETAT DE L'ART

Certaines pathologies comme le diabète nécessitent une surveillance quotidienne de paramètres biochimiques du corps humain, i.e. des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec micro-aiguilles, qui ont l'avantage d'être moins invasive que des aiguilles classiques. Toutefois, il est important que ces micro-aiguilles restent en place. Un dispositif de mesure de l'analyte exemplaire, reposant sur des réseaux de micro-aiguilles, est divulgué dans le document US 2014/275897 A1.

Il existe pour cela des dispositifs à demeure, où des micro-aiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

On connait aussi le dispositif du document WO2018104647, qui présente un boitier comprenant une capsule amovible, la capsule accueillant des microaiguilles configurées pour prélever du liquide interstitiel. Le boitier, quant à lui, accueille la majeure partie de l'électronique.

Toutefois, lorsqu'un tel dispositif comporte des microaiguilles, il peut exister des difficultés liées à l'insertion des micro-aiguilles dans la peau.

L'invention vise à adresser ces difficultés.

### PRESENTATION DE L'INVENTION

Afin de résoudre des difficultés précédemment mentionnées, l'invention propose un capteur pour système de surveillance corporelle, comprenant des microaiguilles de mesure d'analyte configurées pour être insérées dans la peau pour prélever et/ou analyser un fluide corporel du porteur du capteur lorsque ce dernier est positionné sur un membre du porteur,
dans lequel les microaiguilles de mesure d'analyte s'étendent parallèlement à une direction principale depuis un substrat et définissent un plan de travail correspondant au plan passant par la pointe des microaiguilles de mesure de l'analyte, les microaiguilles de mesure d'analyte comprenant chacune sur leur surface un matériau biochimique apte à réagir avec l'analyte,
caractérisé en ce que le capteur comprend au moins une microaiguille distincte de la microaiguille de mesure d'analyte, sur laquelle est montée une électrode de mesure de conductivité configurée pour mesurer le niveau d'enfoncement dans l'épiderme de la microaiguille de mesure d'analyte, l'électrode de mesure de conductivité comprenant une piste métallique, l'extrémité de la piste métallique s'étendant selon la direction principale jusqu'à une position comprise strictement entre le substrat et le plan de travail, les deux étant donc exclus, le capteur comprenant une autre électrode de conductivité, fonctionnant comme une électrode de conductivité de référence, ladite électrode de référence comprenant une piste métallique dont l'extrémité peut atteindre le plan de travail.

Le plan de travail est avantageusement défini par la pointe des microaiguilles de mesure d'analyte ou bien le plan des orifices de prélèvement (dans le cas de microaiguilles qui prélèvent du liquide).

Dans un mode de réalisation, la piste métallique de chaque électrode de conductivité (y compris celle de référence donc) a une longueur inférieure à celle des microaiguilles de mesure biochimique, les deux pistes ayant préférablement la même longueur.

Dans un mode de réalisation, chaque électrode de conductivité est montée sur une microaiguille de conductivité, de longueur égale à celle des microaiguilles de mesure d'analyte.

Dans un mode de réalisation, l'électrode de conductivité est montée sur une microaiguille de conductivité qui n'est que partiellement revêtue de la piste métallique.

Dans un mode de réalisation, la microaiguille de conductivité est intégralement recouverte de métal ou au niveau, préférablement pour l'électrode de référence.

Dans un mode de réalisation, la piste métallique recouvre une portion entière de microaiguille, afin d'augmenter la surface métallique pour une position donnée.

Dans un mode de réalisation, le capteur comprend, réparties sur une ou plusieurs microaiguilles de conductivité, une pluralité d'électrodes de conductivité comprenant chacune une piste métallique s'étendant selon la direction principale jusqu'à au moins deux positions différentes, les positions étant comprises strictement entre le substrat et le plan de travail.

Dans un mode de réalisation, les pistes métalliques sont toutes placées sur une même microaiguille.

Dans un mode de réalisation, la microaiguille a une forme pyramidale tronconique comprenant au moins deux faces et dans lequel les pistes métalliques sont réparties sur plusieurs faces, préférablement une piste par face.

Dans un mode de réalisation, les pistes métalliques sont placées sur des microaiguilles différentes.

Dans un mode de réalisation, les deux positions sont espacées de 200 à 400 µm µm selon la direction principale.

Dans un mode de réalisation, la piste de l'électrode de conductivité de référence a son extrémité a la même position que l'extrémité de la piste métallique de l'électrode de conductivité la plus longue.

Dans un mode de réalisation, la pluralité de pistes métalliques comprend trois pistes métalliques.

Dans un mode de réalisation, l'agencement des électrodes de conductivité est fait de telle sorte que les microaiguilles de mesure d'analyte sont situées entre deux microaiguilles de conductivité.

L'invention propose aussi un système de surveillance corporelle, destiné à être attaché à un membre, comprenant :
- une capsule comprenant un capteur tel que décrit précédemment,
- un boitier, apte à être couplé avec la capsule, à l'intérieur duquel se trouve une batterie et un processeur, le processeur étant configuré pour traiter des données obtenues à l'aide du prélèvement ou de la mesure faite par les microaiguilles, le processeur et la batterie étant configuré pour gérer les mesures de conductivité à l'aide des électrodes.

Le système peut comprendre en outre :
- une lanière ou un bracelet, configuré pour maintenir le boitier en place sur un membre,
- un patch, la capsule étant attachable de façon amovible au patch et le patch étant attachable de façon amovible au boitier, le patch ayant un rôle d'adhésif.

Un autre aspect de l'invention est un procédé de mesure du niveau d'enfoncement dans l'épiderme des microaiguilles de mesure d'analyte d'un capteur conforme à l'invention, comprenant au moins une étape de mesure de la conductivité entre une électrode de conductivité et l'électrode de conductivité de référence.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 illustre en vue éclatée un bracelet, un boitier, une capsule et un patch tel qu'utilisable dans le cadre de l'invention,
- La figure 2 illustre un capteur selon une mode de réalisation de l'invention,
- Les figures 3 à 6 illustrent différentes variantes d'un capteur selon un autre mode de réalisation de l'invention.

### DESCRIPTION DETAILLEE

### Capteur

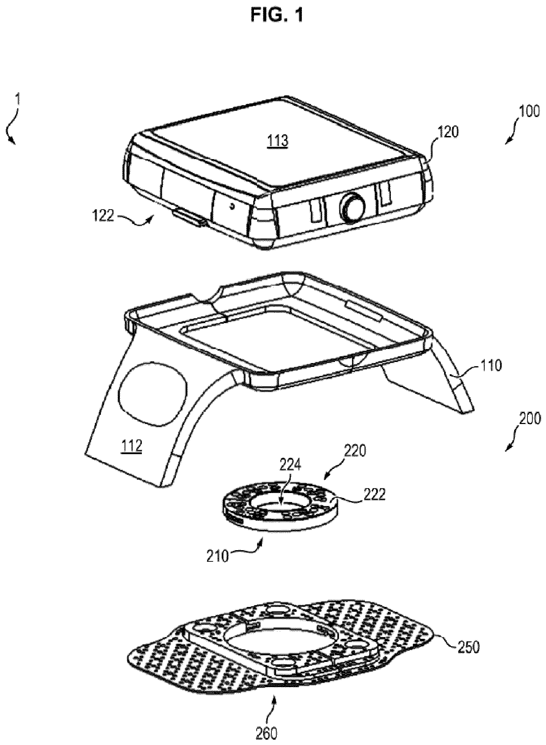

En relation avec les **figures 1 à 6**, un capteur comprenant des microaiguilles de mesure de l'analyte 210 configurées pour être insérées dans la peau pour notamment prélever et/ou analyser un fluide corporel d'un individu va être décrit. Dans le cas de microaiguilles percées formant un canal dans chaque microaiguille, un prélèvement peut être réalisé en connectant de manière fluidique un système de pompage du fluide interstitiel au canal, ou simplement par capillarité. Un système d'analyse peut comprendre des microaiguilles chacune pourvue d'une électrode ou d'un ensemble d'électrodes, ou être déporté après les microaiguilles, de sorte à entraîner une réaction électrochimique adaptée à détecter un analyte dans le fluide interstitiel.

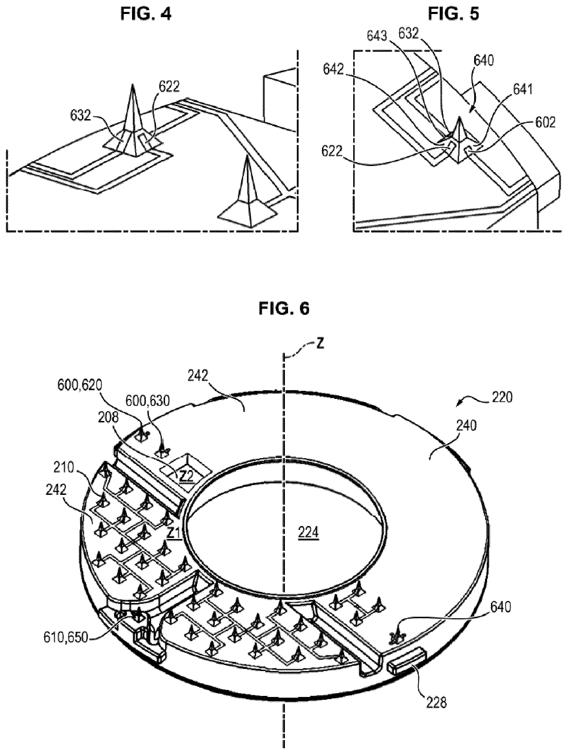

En particulier, le capteur est monté sur une capsule 220 **(****figures 1** **et** **6****)** qui coopère de façon amovible avec un boitier 120 pour former un système de surveillance corporelle 1 attachable à un membre (typiquement un poignet). Ce système sera décrit plus en détail à la fin de la description. La référence 220 s'appliquera aussi bien au capteur qu'à la capsule.

Le capteur 220 comprend une pluralité de microaiguilles de mesure de l'analyte 210. Ces microaiguilles de mesure de l'analyte 210 servent à prélever du liquide ou bien directement à mesurer une grandeur relative à un analyte.

Les microaiguilles de mesure 210 consistent avantageusement en un réseau de microaiguille de mesure 210 au contact de la peau lorsque la capsule 220 est placée sur le corps d'une personne **(****figure 6****)**. Les microaiguilles de mesure de l'analyte 210 peuvent donc être soit creuses, pour prélever du liquide, soit pleines, pour analyser directement le liquide. Dans le premier cas, typiquement, les microaiguilles de mesure de l'analyte 210 permettent l'extraction de liquide interstitiel du derme de façon indolore sans perlement de sang, et l'envoie vers un capteur. Dans le deuxième cas, les microaiguilles de mesure de l'analyte 210 ne prélèvent pas de fluide et intègrent le capteur sur leur surface, sous la forme d'un matériau biochimique apte à réagir avec l'analyte que l'on souhaite mesurer dans le fluide.

De façon préférée, le réseau de microaiguilles de mesure de l'analyte 210 comprend entre quatre et cinquante microaiguilles, sensiblement pyramidales, avec des pointes d'une hauteur comprise entre 100pm et 1000µm, préférablement 0.3mm et 0.8mm. Chacune de ces caractéristiques avantageuses des microaiguilles de mesure de l'analyte 210 peut être prise séparément ou en combinaison avec les autres.

Chaque microaiguille de mesure de l'analyte 210 s'étend depuis un substrat 242 selon une direction principale Z (de façon orthogonale au substrat qui définit une face de contact 222). Ces microaiguilles de mesure 210 définissent un plan de travail Pt : ce plan de travail Pt correspond au plan passant par la pointe de ces microaiguilles de mesure de l'analyte 210 ou bien, alternativement au plan passant par les orifices de prélèvement des microaiguilles de mesure de l'analyte 210, qui se trouvent généralement à l'extrémité ou à proximité de l'extrémité.

S'agissant de microaiguilles de mesure de l'analyte 210, il est primordial de connaître le niveau d'enfoncement dans l'épiderme des microaiguilles de mesure d'analyte 210.

A cet égard, le capteur intègre au moins une électrode de conductivité 600 qui sert à mesurer la conductivité du milieu dans lequel elle se trouve. L'électrode de conductivité 600, qui s'étend depuis le substrat 242, comprend une piste métallique dont l'extrémité s'étend, selon la direction principale Z, jusqu'à une position strictement comprise entre le substrat 242 et le plan de travail Pt des microaiguilles 210 de mesure. Cela signifie, en d'autres termes, que l'électrode de conductivité 600 mesure la conductivité pour du liquide situé entre la pointe des microaiguilles de mesure 210 et l'extérieur de la peau.

Grâce à cette électrode de conductivité 600, il est possible de savoir si les microaiguilles de mesure 210 sont suffisamment enfoncées ou non.

A titre d'exemple, la piste métallique 602 s'étend jusqu'à entre 200 et 400µm à partir du substrat 242 selon la direction principale Z.

Une électrode de conductivité de référence 650, quant à elle, comprend une piste métallique dont l'extrémité peut s'étendre, selon la direction principale Z, jusqu'à une position strictement comprise entre le substrat 242 et le plan de travail Pt des microaiguilles 210 de mesure. Alternativement, l'extrémité peut s'étendre jusqu'au plan de travail Pt.

La mesure de conductivité se fait donc entre l'électrode de conductivité et l'électrode de conductivité de référence.

L'agencement des électrodes de conductivité 600, 650 est fait de telle sorte que les microaiguilles de mesure d'analyte 210 soient situés entre deux microaiguilles de conductivité 600, 650. Dans le cas où plusieurs électrodes 600 travaillent avec une seule électrode de conductivité de référence 650, leur disposition est faite pour toujours avoir des microaiguilles de mesure d'analyte 210 au milieu.

### Electrode et microaiguille

L'électrode de conductivité 600 peut être faite sous la forme d'une microaiguille 604 comprenant une piste métallique 602 s'étendant jusqu'à la position souhaitée. La piste métallique 602 peut être réalisée sur une surface de la microaiguille ou bien être enfouie dans la microaiguille avec seule l'extrémité de la piste métallique 602 qui dépasse. Par conséquent, la position de l'électrode 600 sur la microaiguille 604 n'est pas celle de l'extrémité de la microaiguille 604. De plus, la microaiguille 604 n'est donc que partiellement recouverte de métal.

Les électrodes 600, 650 sont électriquement reliées à un connecteur électrique pour ensuite pouvoir être mise sous tension par le biais d'un module de conductimétrie qui fait appel à un processeur et une batterie. Ces composants seront décrits plus en détail par la suite.

La microaiguille de conductivité a avantageusement la même longueur que les microaiguilles de mesure d'analyte 210 (en s'étendant depuis des zones de substrat faisant partie d'un même plan) afin de simplifier les procédés de fabrication.

La piste métallique peut ne recouvrir que partiellement une portion de la microaiguille ou bien recouvrir toute la microaiguille jusqu'à la hauteur souhaitée (pour améliorer la surface de contact avec l'extérieur - notamment pour les électrodes de conductivité de longueur plus faible que l'électrode de conductivité de référence)

### Premier mode de réalisation

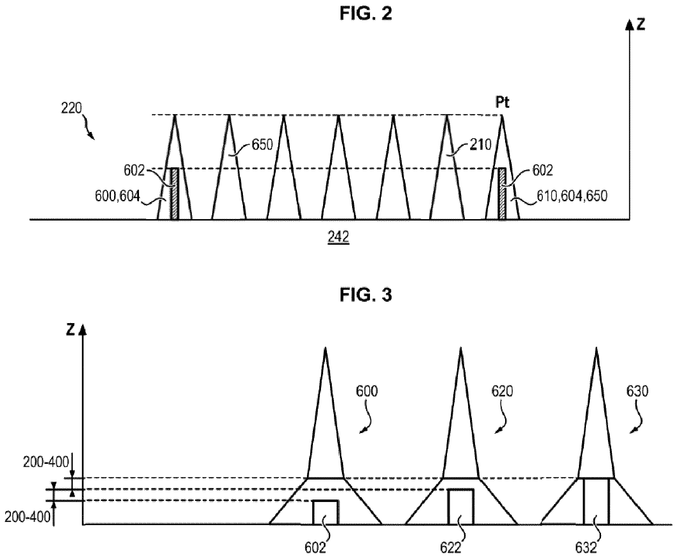

Dans un mode de réalisation **(****figure 2**), une deuxième électrode 610, identique à celle décrite précédemment (qu'on appelle première électrode 600 dans ce cas) est prévue. Cette deuxième électrode 610 correspond à une électrode de conductivité de référence 650 mentionnée précédemment. La mesure de conductivité se fait donc entre ces deux électrodes 600, 610 dont les électrodes sont plus courtes que les microaiguilles 210. Si on définit une droite passant par l'extrémité de ces électrodes (l'extrémité des pistes métalliques 602), celle-ci est parallèle au plan de travail Pt, mais non confondue. Ainsi, un procédé de mesure du niveau d'enfoncement dans l'épiderme des microaiguilles d'analyte 210 peut être mise en oeuvre en mesurant la conductivité entre les deux électrodes de conductivité 600 et 610.

La deuxième électrode de conductivité 610 est préférablement montée sur une microaiguille, de la même façon que cela a été décrit pour la première électrode de conductivité 600.

### Deuxième mode de réalisation

Dans un autre mode de réalisation **(****figures 3 à 6**), plusieurs électrodes de conductivité 600, 620, 630 comprenant chacune une piste métallique 602, 622, 632 qui s'étend depuis le substrat à des positions différentes entre elles selon la direction principale Z. Ces positions sont toutes strictement comprises entre le plan de travail Pt et le substrat 242.

La mesure de conductivité se fait entre une de ces électrodes de conductivité 600, 620, 630 et l'électrode de conductivité de référence 650. Ainsi, un procédé de mesure du niveau d'enfoncement dans l'épiderme des microaiguilles d'analyte 210 peut être mise en oeuvre en mesurant la conductivité entre les électrodes de conductivité 600, 620 et 630.

Ces différentes électrodes de conductivité 600, 620, 630 permettent de connaitre plus précisément le niveau d'enfoncement des microaiguilles 210. En effet, en référence aux **figures 3** **et** **4****,** si l'électrode de conductivité 600 (la plus courte) présentent une conductivité relative à une immersion, alors les microaiguilles 210 sont suffisamment enfoncées. Si en revanche seules les électrodes de conductivité 620, 630 (les plus longues) présentent une conductivité relative à une immersion, alors il y a un risque que l'enfoncement ne soit pas suffisamment. Si seule l'électrode de conductivité 630 présente une conductivité relative à une immersion, alors il y a un risque que les microaiguilles de mesure d'analytes ne soient pas enfoncées suffisamment.

Le principe est généralisable à plus d'électrodes.

Préférablement, l'électrode de conductivité de référence 650 est similaire à l'électrode de conductivité 630 - la plus profonde donc.

La différence de hauteur selon la direction principale Z entre deux pistes métalliques 602, 622, 632 de deux électrodes de conductivité 600, 620, 630 est typiquement comprise entre 50µm et 400µm, préférablement entre 50 et 150µm. Par exemple, s'il y a trois électrodes, on pourra avoir un écart de 200µm entre la première et la deuxième et 200µm entre la deuxième et la troisième. La piste 602 (la plus courte) se trouve typiquement entre 50 et 400µm du substrat 242 selon la direction principale Z.

Les électrodes de conductivité 600, 620, 630 peuvent être montées sur des microaiguilles distinctes, comme illustré en **figure 3****.**

Alternativement, les électrodes 600, 620, 630 peuvent être montées sur une seule microaiguille 640 **(****figures 4 et 5****)**. Dans une variante, les pistes 602, 622, 632 sont présentes sur une même face de la microaiguille 640. Dans une variante préférée, on choisit une microaiguille 640 avec une portion tronconique (par exemple pyramidale) présentant au moins trois faces latérales 641, 642, 643 (voire quatre) : les électrodes sont ainsi disposées sur des faces différentes de la forme tronconique pyramidale.

Lors de chaque mesure de conductivité, chaque électrode de conductivité 630, 640 se comporte donc comme une électrode 600 telle que présentée dans la formulation générale de l'invention, par rapport à l'électrode conductivité de référence 650.

Ces deux alternatives peuvent être montées toutes les deux sur un même capteur, afin d'avoir une certaine redondance. La **figure 6** illustre cela,

### Architecture générale

En référence à la **figure 1**, qui en représente un schéma général, la présente invention concerne un système électronique 1 de surveillance corporelle.

Par surveillance corporelle, on entend la vérification de constantes biochimiques d'une personne porteuse du système 1, typiquement la concentration en une protéine, une hormone, un marqueur, en oxygène, en nutriments, etc., dans le liquide interstitiel de la personne. On citera l'exemple de la glycémie. L'homme du métier pourra surveiller si besoin d'autres grandeurs physiques corporelles telles que le lactate, l'hydratation, etc.

La description sera illustrée avec du liquide interstitiel mais s'applique aux autres liquides corporels tels que le sang.

Le système 1 est dit autonome, car il ne nécessite pas l'utilisation de matériel supplémentaire.

Le système 1 est formé de deux modules 100, 200 **(****figure 1**) reliées entre eux par une liaison séparable 300 qui est réutilisable.

Le premier module 100 comprend notamment des moyens d'attache et de serrage 110 du système 1 à un membre (qu'on appellera lanière ou bracelet 112) et le deuxième module 200 comprend une capsule 220 qui le capteur décrit précédemment.

Les deux modules 100 et 200 présentent chacun une face de couplage 122, 222, de forme complémentaire, qui permet de placer le deuxième module 200 dans un emplacement d'accueil du premier module 100

Enfin, le premier module 100 et le deuxième module 200 sont configurés pour être couplés par une liaison séparable 300.

Comme illustré sur la **figure 1**, le premier module 100 comprend en outre un boitier 120 dans lequel sont disposés des moyens de traitement de données (en particulier un processeur ou un microcontrôleur) configurés pour traiter des mesures acquises par le capteur, et le cas échéant des moyens de stockage de données (notamment une mémoire, en particulier de type flash, et/ou la mémoire du microcontrôleur) permettant par exemple de stocker ces mesures, et/ou une date de la première utilisation de chaque capteur pour calculer une date de péremption du ou des capteurs (les capteurs biochimiques ont une durée de vie limitée). Les moyens de traitement de données servent aussi à générer des consignes vers différentes composants. Dans le cadre de cette description, ces différentes fonctions sont assurées par une même unité. Toutefois, il est possible de prévoir des processeurs dédiés. Le système comprend également une batterie pour l'alimentation électrique des composants, avantageusement rechargeable, par exemple via un port (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran, éventuellement tactile pour afficher les résultats de la surveillance à l'utilisateur.

L'homme du métier connait des algorithmes de traitement de mesures de capteurs 24 et des interfaces associées, et saura les implémenter dans le présent système 1.

Le boitier 120 comprend en outre des connecteurs électriques, sur sa face de couplage 122 avec la face de couplage 222 de la capsule 220.

La capsule 220 du deuxième module 200 a une forme de boite fermée, étanche, qui peut se coupler avec le boitier 120 100. Cette capsule 220 est interchangeable, ce qui permet d'obtenir un système économique et efficace, où seules les parties dites consommables doivent être changées. La capsule 220 peut avoir une forme annulaire, avec une ouverture traversante 224 au centre. Dans une variante mentionnée précédemment, le capteur est positionné à l'intérieur de la capsule 220 (ou alors dans le boitier 120) et analyse le fluide prélevé par les microaiguilles. La capsule 220 comprend des connecteurs électriques 226, sur une face de couplage 222 avec le boitier 120, qui peuvent coopèrent avec les connecteurs électriques du boitier 120. S'il y a transmission de fluide depuis le deuxième module vers le premier alors des connecteurs fluidiques complémentaires sont prévus sur la capsule 220 et le boitier 120.

Enfin, le deuxième module 200 comprend un patch 250, solidaire de façon amovible à la capsule 220, qui est décrit en détail ci-dessous.

Le deuxième module 200 (capsule 220 et patch 250) forme un ensemble interchangeable du système qui est choisi selon le type de surveillance voulu et en fonction de l'état de détérioration des microaiguilles 210 et/ou du capteur.

En effet, dans la mesure où la capsule 220 contient les microaiguilles 210 et/ou le capteur (notamment dans le cas de microaiguilles qui prélèvent), changer de capsule 220 permet de changer de matériel si celui-ci est en fin de vie ou si l'on souhaite changer de grandeur physique mesurée, en une manipulation simple, rapide et sure, sans devoir jeter d'autres parties (en particulier le premier module 100).

Et dans la mesure où la capsule 220 minimise la quantité d'élément et/ou matériaux coûteux (équipement électronique avancé tel qu'une batterie ou des moyens de communication sans fil), elle est relativement peu chère.

## Revendications

1. Capteur (220) pour système de surveillance corporelle (1), comprenant des microaiguilles de mesure d'analyte (210) configurées pour être insérées dans la peau pour prélever et/ou analyser un fluide corporel du porteur du capteur lorsque ce dernier est positionné sur un membre du porteur,
dans lequel les microaiguilles de mesure d'analyte (210) s'étendent parallèlement à une direction principale (Z) depuis un substrat (242), définissent un plan de travail (Pt) correspondant au plan passant par la pointe des microaiguilles de mesure de l'analyte (210), les microaiguilles de mesure d'analyte (210) comprenant chacune sur leur surface un matériau biochimique apte à réagir avec l'analyte,
**caractérisé en ce que** le capteur (220) comprend au moins une microaiguille distincte de la microaiguille de mesure d'analyte, sur laquelle est montée une électrode de mesure de conductivité (600, 610, 620, 630), l'électrode de mesure de conductivité (600, 610, 620, 630) comprenant une piste métallique (602), l'extrémité de la piste métallique s'étendant selon la direction principale (Z) jusqu'à une position comprise strictement entre le substrat (242) et le plan de travail (Pt), les deux étant donc exclus,
le capteur (220) comprenant une autre électrode de conductivité , fonctionnant comme une électrode de conductivité de référence (650), ladite électrode de conductivité de référence (650) comprenant une piste métallique (602) dont l'extrémité peut atteindre le plan de travail (Pt), la conductivité mesurée entre l'électrode de conductivité (600) et l'électrode de conductivité de référence (650) étant caractéristique du niveau d'enfoncement dans l'épiderme de la microaiguille de mesure d'analyte (210).

2. Capteur (220) selon la revendication 1, dans lequel la piste métallique (602) de chaque électrode de conductivité (600, 610, 620, 630) a une longueur inférieure à celle des microaiguilles de mesure d'analyte (210), les deux pistes ayant préférablement la même longueur.

3. Capteur (220) selon l'une quelconque des revendications précédentes, dans lequel chaque électrode de conductivité (600, 610, 620, 630) est montée sur une microaiguille de conductivité (604), de longueur égale à celle des microaiguilles de mesure d'analyte 210.

4. Capteur (220) selon l'une quelconque des revendications précédentes, dans lequel l'électrode de conductivité (600, 610, 620, 630, 650) est montée sur une microaiguille de conductivité qui n'est que partiellement revêtue de la piste métallique.

5. Capteur (220) selon la revendication 1, comprenant, réparties sur une ou plusieurs microaiguilles de conductivité, une pluralité d'électrodes de conductivité (600, 610, 620, 630) comprenant chacune une piste métallique (602, 622, 632) s'étendant selon la direction principale (Z) jusqu'à au moins deux positions différentes, les positions étant comprises strictement entre le substrat (242) et le plan de travail (Pt).

6. Capteur (220) selon la revendication 5, dans lequel les pistes métalliques (602, 622, 632) sont toutes placées sur une même microaiguille (640).

7. Capteur (220) selon la revendication 6, dans lequel la microaiguille (640) a une forme pyramidale tronconique comprenant au moins deux faces (641, 642, 643) et dans lequel les pistes métalliques (602, 622, 632) sont réparties sur plusieurs faces, préférablement une piste par face.

8. Capteur (220) selon la revendication 7, dans laquelle les pistes métalliques (602, 622, 632) sont placés sur des microaiguilles différentes.

9. Capteur (220) selon l'une quelconque des revendications 5 à 8, dans lequel les deux positions sont espacées de 200 à 400 µm selon la direction principale (Z).

10. Capteur (220) selon l'une quelconque des revendications 5 à 9, dans lequel la piste (602) de l'électrode de conductivité de référence (650) a son extrémité a la même position que l'extrémité de la piste métallique (602, 622, 632) de l'électrode de conductivité (600, 620, 630) la plus longue.

11. Capteur (220) selon l'une des revendications 1 à 10, dans lequel l'agencement des électrodes de conductivité (600, 650) est fait de telle sorte que les microaiguilles de mesure d'analyte (210) sont situées entre deux électrodes de conductivité (600, 650).

12. Système de surveillance corporelle (1), destiné à être attaché à un membre, comprenant :
- une capsule (220) comprenant un capteur selon l'une quelconque des revendications 1 à 11,
- un boitier (120), apte à être couplé avec la capsule (220), à l'intérieur duquel se trouve une batterie et un processeur, le processeur étant configuré pour traiter des données obtenues à l'aide du prélèvement ou de la mesure faite par les microaiguilles (210), le processeur et la batterie étant configuré pour gérer les mesures de conductivité à l'aide des électrodes (600, 610, 620, 630).

13. Système de surveillance corporelle (1) selon la revendication 12, comprenant en outre :
- une lanière ou un bracelet, configuré pour maintenir le boitier (120) en place sur un membre,
- un patch (250), la capsule (220) étant attachable de façon amovible au patch (250) et le patch (250) étant attachable de façon amovible au boitier (120), le patch (250) ayant un rôle d'adhésif.

14. Procédé de mesure du niveau d'enfoncement dans l'épiderme des microaiguilles de mesure d'analyte (210) d'un capteur conforme à l'une des revendications 1 à 11, comprenant au moins une étape de mesure de la conductivité entre une électrode de conductivité (600) et l'électrode de conductivité de référence (650).

## Patentansprüche

1. Sensor (220) für ein Körperüberwachungssystem (1), der Mikronadeln zur Analytmessung (210) umfasst, die so konfiguriert sind, dass sie in die Haut eingeführt werden, um Körperflüssigkeit von dem Träger des Sensors zu entnehmen und/oder zu analysieren, wenn letzterer an einem Körperglied des Trägers positioniert ist,
wobei sich die Mikronadeln zur Analytmessung (210) von einem Substrat (242) aus parallel zu einer Hauptrichtung (Z) erstrecken, eine Arbeitsebene (Pt) definieren, die der Ebene entspricht, die durch die Spitze der Mikronadeln zur Analytmessung (210) verläuft, wobei die Mikronadeln zur Analytmessung (210) jeweils auf ihrer Oberfläche ein biochemisches Material aufweisen, das mit dem Analyten reagieren kann,
**dadurch gekennzeichnet, dass** der Sensor (220) mindestens eine von der Mikronadel zur Analytmessung getrennte Mikronadel umfasst, an der eine Elektrode zur Leitfähigkeitsmessung (600, 610, 620, 630) angebracht ist, wobei die Elektrode zur Leitfähigkeitsmessung (600, 610, 620, 630) eine Metallbahn (602) umfasst, wobei sich das Ende der Metallbahn in der Hauptrichtung (Z) bis zu einer Position erstreckt, die strikt zwischen dem Substrat (242) und der Arbeitsebene (Pt) liegt, wobei beide somit ausgeschlossen sind,
wobei der Sensor (220) eine weitere Leitfähigkeitselektrode umfasst, die als Referenz-Leitfähigkeitselektrode (650) fungiert, wobei die Referenz-Leitfähigkeitselektrode (650) eine Metallbahn (602) umfasst, deren Ende die Arbeitsebene (Pt) erreichen kann, wobei die zwischen der Leitfähigkeitselektrode (600) und der Referenz-Leitfähigkeitselektrode (650) gemessene Leitfähigkeit charakteristisch für den Grad des Eindringens der Mikronadel zur Analytmessung (210) in die Epidermis ist.

2. Sensor (220) nach Anspruch 1, wobei die Metallbahn (602) jeder Leitfähigkeitselektrode (600, 610, 620, 630) eine geringere Länge als diejenige der Mikronadeln zur Analytmessung (210) aufweist, wobei die beiden Bahnen vorzugsweise die gleiche Länge aufweisen.

3. Sensor (220) nach einem der vorstehenden Ansprüche, wobei jede Leitfähigkeitselektrode (600, 610, 620, 630) an einer Leitfähigkeitsmikronadel (604) angebracht ist, deren Länge derjenigen der Mikronadeln zur Analytmessung (210) entspricht.

4. Sensor (220) nach einem der vorstehenden Ansprüche, wobei die Leitfähigkeitselektrode (600, 610, 620, 630, 650) an einer Leitfähigkeitsmikronadel angebracht ist, die nur teilweise mit der Metallbahn bedeckt ist.

5. Sensor (220) nach Anspruch 1, der auf eine oder mehrere Leitfähigkeitsmikronadeln verteilt eine Vielzahl von Leitfähigkeitselektroden (600, 610, 620, 630) umfasst, von denen jede eine Metallbahn (602, 622, 632) umfasst, die sich in der Hauptrichtung (Z) bis zu mindestens zwei verschiedenen Positionen erstreckt, wobei die Positionen strikt zwischen dem Substrat (242) und der Arbeitsebene (Pt) liegen.

6. Sensor (220) nach Anspruch 5, wobei die Metallbahnen (602, 622, 632) alle auf einer einzigen Mikronadel (640) angeordnet sind.

7. Sensor (220) nach Anspruch 6, wobei die Mikronadel (640) eine kegelstumpfförmige Pyramidenform mit mindestens zwei Seitenflächen (641, 642, 643) aufweist und wobei die Metallbahnen (602, 622, 632) auf mehrere Seitenflächen verteilt sind, vorzugsweise eine Bahn pro Seitenfläche.

8. Sensor (220) nach Anspruch 7, wobei die Metallbahnen (602, 622, 632) auf verschiedenen Mikronadeln angeordnet sind.

9. Sensor (220) nach einem der Ansprüche 5 bis 8, wobei die beiden Positionen in der Hauptrichtung (Z) um 200 bis 400 µm voneinander beabstandet sind.

10. Sensor (220) nach einem der Ansprüche 5 bis 9, wobei die Bahn (602) der Referenz-Leitfähigkeitselektrode (650) ihr Ende an derselben Position hat wie das Ende der Metallbahn (602, 622, 632) der längsten Leitfähigkeitselektrode (600, 620, 630).

11. Sensor (220) nach einem der Ansprüche 1 bis 10, wobei die Anordnung der Leitfähigkeitselektroden (600, 650) so erfolgt, dass sich die Mikronadeln zur Analytmessung (210) zwischen zwei Leitfähigkeitselektroden (600, 650) befinden.

12. Körperüberwachungssystem (1), das dafür bestimmt ist, an einem Körperglied befestigt zu werden, umfassend:
- eine Kapsel (220), die einen Sensor nach einem der Ansprüche 1 bis 11 umfasst,
- ein Gehäuse (120), das mit der Kapsel (220) gekoppelt werden kann und in dem sich eine Batterie und ein Prozessor befinden, wobei der Prozessor so konfiguriert ist, dass er Daten verarbeitet, die mithilfe der durch die Mikronadeln (210) vorgenommenen Entnahme oder Messung erhalten wurden, wobei der Prozessor und die Batterie so konfiguriert sind, dass sie die Leitfähigkeitsmessungen mithilfe der Elektroden (600, 610, 620, 630) verwalten.

13. Körperüberwachungssystem (1) nach Anspruch 12, das ferner umfasst:
- einen Riemen oder ein Armband, der/das so konfiguriert ist, dass er/es das Gehäuse (120) an einem Körperglied festhält,
- ein Pflaster (250), wobei die Kapsel (220) lösbar an dem Pflaster (250) befestigt werden kann und das Pflaster (250) lösbar an dem Gehäuse (120) befestigt werden kann, wobei das Pflaster (250) eine klebende Funktion hat.

14. Verfahren zur Messung des Grades des Eindringens der Mikronadeln zur Analytmessung (210) eines Sensors nach einem der Ansprüche 1 bis 11 in die Epidermis, umfassend mindestens einen Schritt der Messung der Leitfähigkeit zwischen einer Leitfähigkeitselektrode (600) und der Referenz-Leitfähigkeitselektrode (650).

## Claims

1. A sensor (220) for a body monitoring system (1), comprising analyte measuring microneedles (210) configured to be inserted into the skin to sample and/or analyse a body fluid from the wearer of the sensor when the sensor is positioned on a limb of the wearer,
in which the analyte-measuring microneedles (210) extend parallel to a main direction (Z) from a substrate (242), define a working plane (Pt) corresponding to the plane passing through the tip of the analyte-measuring microneedles (210), the analyte-measuring microneedles (210) each comprising on their surface a biochemical material capable of reacting with the analyte,
**characterised in that** the sensor (220) comprises at least one microneedle separate from the analyte-measuring microneedle, on which is mounted a conductivity electrode (600, 610, 620, 630), the conductivity-measuring electrode (600, 610, 620, 630) comprising a metal track (602), the end of the metal track extending in the main direction (Z) to a position strictly between the substrate (242) and the working plane (Pt),
the sensor (220) comprising another conductivity electrode, functioning as a reference conductivity electrode (650), said reference conductivity electrode (650) comprising a metal track (602) whose end can reach the working plane (Pt), the conductivity measured between the conductivity electrode (600) and the reference conductivity electrode (650) being characteristic of the level of penetration into the epidermis of the analyte-measuring microneedle (210).

2. A sensor (220) as claimed in claim 1, wherein the metal track (602) of each conductivity electrode (600, 610, 620, 630) has a length smaller than that of the analyte measuring microneedles (210), the two tracks preferably being of the same length.

3. A sensor (220) according to any one of the preceding claims, in which each conductivity electrode (600, 610, 620, 630) is mounted on a conductivity microneedle (604) of a length equal to that of the analyte-measuring microneedles (210).

4. A sensor (220) according to any one of the preceding claims, wherein the conductivity electrode (600, 610, 620, 630, 650) is mounted on a conductivity microneedle which is only partially coated with the metal track.

5. A sensor (220) as claimed in claim 1, comprising, distributed over one or more conductivity microneedles, a plurality of conductivity electrodes (600, 610, 620, 630) each comprising a metal track (602, 622, 632) extending in the main direction (Z) to at least two different positions, the positions lying strictly between the substrate (242) and the working plane (Pt).

6. A sensor (220) according to claim 5, wherein the metal tracks (602, 622, 632) are all placed on the same microneedle (640).

7. Sensor (220) according to claim 6, in which the microneedle (640) has a truncated pyramidal shape comprising at least two faces (641, 642, 643) and in which the metal tracks (602, 622, 632) are distributed over several faces, preferably one track per face.

8. Sensor (220) according to claim 7, in which the metal tracks (602, 622, 632) are placed on different microneedles.

9. Sensor (220) according to any one of claims 5 to 8, in which the two positions are spaced apart by 200 to 400 µm in the main direction (Z).

10. A sensor (220) according to any one of claims 5 to 9, wherein the track (602) of the reference conductivity electrode (650) has its end in the same position as the end of the metal track (602, 622, 632) of the longer conductivity electrode (600, 620, 630).

11. Sensor (220) according to one of claims1 to 10, in which the arrangement of the conductivity electrodes (600, 650) is such that the analyte-measuring microneedles (210) are located between two conductivity electrodes (600, 650).

12. A body monitoring system (1), for attachment to a limb, comprising:
- a capsule (220) comprising a sensor according to any one of claims 1 to 11,
- a case (120), suitable for being coupled with the capsule (220), inside which is a battery and a processor, the processor being configured to process data obtained using the sampling or measurement made by the microneedles (210), the processor and the battery being configured to manage the conductivity measurements using the electrodes (600, 610, 620, 630).

13. A body monitoring system (1) according to claim 12, further comprising:
- a strap or bracelet, configured to hold the case (120) in place on a limb,
- a patch (250), the capsule (220) being removably attachable to the patch (250) and the patch (250) being removably attachable to the case (120), the patch (250) acting as an adhesive.

14. Method of measuring the level of penetration into the epidermis of the analyte-measuring microneedles (210) of a sensor according to one of claims 1 to 11, comprising at least one step of measuring the conductivity between a conductivity electrode (600) and the reference conductivity electrode (650).
